# EUROPEAN PATENT SPECIFICATION

(11) **EP 4 026 140 B1**
(45) Date of publication and mention of the grant of the patent: **30.10.2024**
(21) Application number: 20807301.5
(22) Date of filing: 10.11.2020
(51) Int. Cl.: G16H 30/20

(54) **ADJUSTMENT METHOD FOR AN ANALYTICAL DETERMINATION OF AN ANALYTE IN A BODILY FLUID**
EINSTELLUNGSVERFAHREN ZUR ANALYTISCHEN BESTIMMUNG EINES ANALYTEN IN EINER KÖRPERFLÜSSIGKEIT
PROCÉDÉ DE RÉGLAGE D'UNE DÉTERMINATION D'ANALYSE D'UN ANALYTE DANS UN FLUIDE CORPOREL

(30) Priority: 13.11.2019 US 201962934576 P; 22.01.2020 EP 20153112
(43) Date of publication of application: 13.07.2022
(73) Proprietor: F. Hoffmann-La Roche AG, 4070 Basel (CH); Roche Diabetes Care GmbH, 68305 Mannheim (DE)
(72) Inventor: AYSIN, Benhur, Indianapolis, 46250 Indiana (US); BERG, Max, 68305 Mannheim (DE); CHITTAJALLU, Siva, Indianapolis, 46250 Indiana (US); LIMBURG, Bernd, 68305 Mannheim (DE)
(74) Representative: Altmann Stößel Dick Patentanwälte PartG mbB
(86) International application number: PCT/EP2020/081560
(87) International publication number: WO 2021/094274

(56) References cited:
- WO-A1-2014/113770
- WO-A1-2014/113770
- WO-A2-2014/025415
- US-A1- 2014 072 189
- US-A1- 2016 104 057

## Description

### Technical Field

The present invention refers to an adjustment method of adjusting a measurement setup for an analytical method of determining a concentration of an analyte in a bodily fluid, as well as to an adjustment system. The invention further relates to an analytical method of determining a concentration of an analyte in a bodily fluid and to a mobile device configured for performing the analytical method. Further, the present invention refers to a computer program and a computer-readable storage medium for performing the analytical method and to a computer program and a computer-readable storage medium for performing the adjustment method. The methods, devices, computer programs and storage media specifically may be used in medical diagnostics, in order to for example qualitatively or quantitatively detect one or more analytes in one or more body fluids, such as for detecting glucose in blood and/or interstitial fluid. Other fields of application of the present invention, however, are feasible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements comprising one or more test chemicals, which, in presence of the analyte to be detected, are capable of performing one or more detectable detection reactions, such as optically detectable detection reactions. As an example, EP 0 821 234 A2 describes a diagnostic test carrier for the determination of an analyte from whole blood with the aid of a reagent system contained in the carrier and a method for the determination of an analyte from whole blood with the aid of the diagnostic test carrier. The diagnostic test carrier includes a color forming reagent. The test field has a sample application side to which the blood sample is delivered and a detection side where an optically detectable change occurs as a result of the reaction of the analyte with the reagent system. Furthermore, the test field is designed so that the erythrocytes contained in the sample do not reach the detection side. In addition, the test field comprises a transparent film and a first and a second superposed film layer applied thereto, wherein the first layer on the transparent film is substantially less light-scattering in the wet state than the overlying second layer.

With regard to the test chemicals comprised in test elements, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

In analytical measurements, specifically analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers has become more and more popular over the recent years. WO 2012/131386 A1 discloses a testing apparatus for performing an assay, the testing apparatus comprising: a receptacle containing a reagent, the reagent being reactive to an applied test sample by developing a color or pattern variation; a portable device, e.g. a mobile phone or a laptop, comprising a processor and an image capture device, wherein the processor is configured to process data captured by the image capture device and output a test result for the applied test sample.

As opposed to laboratory measurements and measurements performed by using dedicated analytical measurement devices, when using mobile computing devices such as smart phones, various influences need to be taken into account. Many product care activities like improving the measurement flow, improving the customer guidance, designing and optimizing failsafe algorithms or optimizing measurement performance are often based either on extensive market research or complaint devices coming back from the market. Extensive market research, however, may be expensive and not continuously given. Further, it might be preferable to optimize measurement algorithms and setups in advance, prior to receiving customers' complaints.

In the field of technology of mobile computing devices, various technical approaches have been developed over the recent years in order to improve image recognition and in order to gain additional information regarding, for example, unknown geometrical parameters of the setup.

US 9750433 B2 discloses a method for communicating activity-related notifications to a user. The method includes: receiving a record of activity events of a particular activity type performed by the user over a period of time; selecting a first time-based filter from a set of time-based filters; identifying a cluster of activity events in the record of activity events filtered according to the first time-based filter; identifying an early bound and a late bound on start times of activity events of the particular activity type from the cluster; communicating a notification of a first type to the user at a first time within a threshold time of the early bound on a day fulfilling the first time-based filter; and communicating a notification of a second type to the user at a second time within a threshold time of the late bound on a day fulfilling the first time-based filter.

US 10347152 B2 discloses a health and fitness management system that employs an algorithm to determine suggested recommended actions for a user to improve their health and fitness. The system obtains a user's weight from a scale. The user is never informed of their weight. Other data can be collected and included when calculating a health index number. Base line data, such as age, ideal age, initial weight, current weight, ideal weight, etc. can be considered in the algorithm. Examples include the user's environment, sleep habits, exercise routines, medical records, and the like. The health index number is used to determine recommended actions, which can include changes to environments, routines, activities, etc. Data collection, the algorithm, and other features of the system can be provided by an Application operating on a portable computing device. Features of the portable computing device can be employed to automatically acquire data for the algorithm.

US 6454708 B1 discloses a system and a method for monitoring health parameters and capturing data from a subject. The system is characterized by a cordless, disposable sensor band with sensors for measuring full waveform ECG, full waveform respiration, skin temperature, and motion, and a connector which accepts a memory card or a smart card for storage of the measured data. After a predetermined period of time, such as when the sensor band is removed, the memory card or smart card is removed and inserted into a monitoring device which reads the stored health parameter data of the subject. The monitoring device includes a base station that includes a memory/smart card reader and is connected to conventional phone lines for transferring the collected data to a remote monitoring station. The base station may also capture additional clinical data, such as blood pressure data, and to perform data checks. Subject safety is enhanced by the ability of the base station to compare clinical data, e.g. ECG, against given profiles and to mark events when appropriate or when the base station is programmed to do so. The remote monitoring station allows the presentation and review of data (including events) forwarded by the sensor band. ECG analysis software and a user-friendly graphical user interface are provided to remotely analyze the transmitted data and to permit system maintenance and upkeep. In alternative embodiments, a smart card includes the sensor band's electronics and/or signal transmission circuitry in conjunction with a portable data logger so that the electronics may be reused from one disposable sensor band to the next without limiting the patient's range of movement. The system of the invention has useful application to the collection of subject clinical data during drug trials and medical testing for regulatory approvals as well as management of subjects with chronic diseases.

US 20150294576 A1 discloses a nutrition and activity management system that monitors energy expenditure of an individual through the use of a body-mounted sensing apparatus. The apparatus is particularly adapted for continuous wear. The system is also adaptable or applicable to measuring a number of other physiological parameters and reporting the same and derivations of such parameters. A weight management embodiment is directed to achieving an optimum or preselected energy balance between calories consumed and energy expended by the user. An adaptable computerized nutritional tracking system is utilized to obtain data regarding food consumed, Relevant and predictive feedback is provided to the user regarding the mutual effect of the user's energy expenditure, food consumption and other measured or derived or manually input physiological contextual parameters upon progress toward said goal.

US 10178973 B2 discloses the use of a biometric monitoring device to determine a user's heart rate by using a heartbeat waveform sensor and a motion detecting sensor. In some embodiments, the device collects output data from the heartbeat waveform sensor and output data from the motion detecting sensor, determines an expected heart rate range from the output data from the motion detecting sensor, and determines the user's heart rate within the expected heart rate range using the output data from the heartbeat waveform sensor.

US 20160328991 A1 discloses systems and methods by which users may learn and become familiar with the effects of various aspects of their lifestyle on their health, e.g., users may learn about how food and/or exercise affects their glucose level and other physiological parameters, as well as overall health. In some cases the user selects a program to try; in other cases, a computing environment embodying the system suggests programs to try, including on the basis of pattern recognition, i.e., by the computing environment determining how a user could improve a detected pattern in some way. In this way, users such as type II diabetics or even users who are only prediabetic or non-diabetic may learn healthy habits to benefit their health.

US 2016/0104057 A1 discloses a method of operating a computing device to learn user preferences of how to process digital images. The method can include: aggregating a user image selection and a context attribute associated therewith into a preference training database for a user, wherein the user image selection represents a record of the user's preference over at least one of adjusted versions of a base image when the adjusted versions are separately processed by different visual effects; determining a visual effect preference associated based on machine learning or statistical analysis of user image selections in the preference training database, the user image selections representing experimental records corresponding to the visual effects; updating a photo preference profile with the visual effect preference; and providing the photo preference profile to an image processor to adjust subsequently captured photographs provided to the image processor.

WO 2014/113770 A1 is the closest prior art and discloses a method for obtaining a point-of-collection, selected quantitative indicia of an analyte on a test strip using a smartphone involving imaging a test strip on which a colorimetric reaction of a target sample has occurred due to test strip illumination by the smartphone. The smartphone includes a smartphone app and a smartphone accessory that provides an external environment-independent/intemal light-free, imaging environment independent of the smartphone platform being used. The result can then be presented quantitatively or turned into another measurement (positive, negative, above average, etc.), displayed to the user, stored for later use, and communicated to a location where practitioners can provide additional review. Additionally, social media integration can allow for device results to be broadcast to specific audiences, to compare healthy living with others, to compete in health based games, create mappings, and other applications.

Despite the advantages achieved by the known methods and devices, specifically in the field of mobile computing, several technical challenges remain. Specifically, reliability and accuracy of the measurements need to be enhanced and ensured. Further, a major challenge still relies in the fact that user-specific preferences and user-specific handling of measurement devices and measurement processes differ to a wide extent. This challenge is mainly due to the fact that a wide variety of users is intended to apply the algorithms and measurement setups, having differing physical or mental capabilities and preferences.

### Problem to be solved

It is therefore desirable to provide methods and devices which address the above-mentioned technical challenges of analytical measurements using mobile devices such as consumer-electronics mobile devices, specifically multipurpose mobile devices which are not dedicated to analytical measurements such as smart phones or tablet computers. Specifically, methods and devices shall be proposed which take into account user-specific preferences and capabilities and which, thus, automatically may adapt to the specific circumstances of measurement, by still using standard mobile devices without the necessity of manually adapting device settings.

### Summary

This problem is addressed by the methods, devices, computer programs and computer-readable storage media with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, an adjustment method is disclosed. The adjustment method is configured for adjusting a measurement setup, the measurement setup being configured for performing an analytical method of determining a concentration of an analyte in a bodily fluid. Therein, the analytical method comprises using a mobile device having a camera, capturing at least one image of at least a part of an optical test strip having a test field by using the camera, and further determining at least one analyte concentration value from color formation of the test field. The adjustment method comprises the following steps which specifically may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed. The adjustment method comprises:
i) carrying out, by a plurality of users, a plurality of analyte measurements, wherein the analyte measurements, at least partly, comprise capturing images of at least a part of an optical test strip having a test field by using the camera, thereby obtaining training data on the analyte measurements;
ii) analyzing the training data obtained in step i), thereby identifying similarities in the training data and identifying a plurality of user profiles according to the similarities in the training data; and
iii) providing profile-specific measurement setup adjustments for at least one of the user profiles.

The term "analytical method of determining the concentration of an analyte in a bodily fluid", also simply referred to as an "analytical method" or an "analytical measurement", as used herein is a broad term and is to be given its ordinary and customary meaning to a person of ordinary skill in the art and is not to be limited to a special or customized meaning. The term specifically may refer, without limitation, to a quantitative and/or qualitative determination of at least one analyte in an arbitrary sample or aliquot of bodily fluid. For example, the bodily fluid may comprise one or more of blood, interstitial fluid, urine, saliva or other types of body fluids. The result of the determining of the concentration, as an example, may be a concentration of the analyte and/or the presence or absence of the analyte to be determined. Specifically, as an example, the analytical measurement may be a blood glucose measurement, thus the result of the analytical measurement may for example be a blood glucose concentration. In particular, an analytical measurement result value may be determined by the analytical measurement. Consequently, the term "analytical measurement result value", also referred to as an "analyte concentration value" or "analytical measurement result value", as used herein, is a broad term and is to be given its ordinary and customary meaning to a person of ordinary skill in the art and is not to be limited to a special or customized meaning. The term specifically may refer, without limitation, to a numerical indication of an analyte concentration in a sample. The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value.

The term "measurement setup for an analytical method of determining a concentration of an analyte in a bodily fluid" as used herein is a broad term and is to be given its ordinary and customary meaning to a person of ordinary skill in the art and is not to be limited to a special or customized meaning. The term specifically may refer, without limitation, to one or more of a system, a device, a configuration of a system or a configuration of a device for performing the analytical method. Specifically, the term may refer to the combination of at least one device, such as a mobile device, and at least one software configured for running at least one application on the device, such as at least one software for performing the analytical measurement. The measurement setup may refer to, as an example, one or more of a user-specific setup, a handling-specific setup, a setup specific for a measurement environment or the like. Generally, the term measurement setup may thus refer to the way of performing the analytical method, such as one or more of the at least one device used for performing the analytical method, handling steps for performing the analytical method or the user performing the analytical method.

Consequently, the term "adjustment method of adjusting a measurement setup for an analytical method of determining a concentration of an analyte in a bodily fluid", also simply referred to as an "adjustment method", as used herein also is a broad term and is to be given its ordinary and customary meaning to a person of ordinary skill in the art and is not to be limited to a special or customized meaning. The term specifically may refer, without limitation, to a method of one or more of changing or adjusting the measurement setup as defined above. Specifically, the term may refer to a method by which at least one parameter having an influence on the measurement setup is adjusted and/or set. Exemplary embodiments will be given below.

The analytical method, as outlined above, comprises using a mobile device having a camera. The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, as will be outlined in further detail below, the mobile device may also refer to a tablet computer or another type of portable computer having at least one camera.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. a larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

The analytical method further comprises capturing at least one image of at least a part of an optical test strip having a test field by using the camera. The term "optical test strip" as used herein is a broad term and is to be given its ordinary and customary meaning to a person of ordinary skill in the art and is not to be limited to a special or customized meaning. The term specifically may refer, without limitation, to an arbitrary element or device configured for performing a color-change detection reaction. The optical test strip may also be referred to as test strip or test element, wherein all three terms may refer to the same element. The optical test strip may particularly have a test field containing at least one test chemical for detecting at least one analyte. The optical test strip, as an example, may comprise at least one substrate, such as at least one carrier, with the at least one test field applied thereto or integrated therein. In particular, the optical test strip may further comprise at least one white area, such as a white field, specifically in a proximity to the test field, for example enclosing or surrounding the test field. The white area may be a separate field independently arranged on the substrate or carrier. However, additionally or alternatively, the substrate or carrier itself may be or may comprise the white area. As an example, the at least one carrier may be strip-shaped, thereby rendering the test element a test strip. These test strips are generally widely in use and available. One test strip may carry a single test field or a plurality of test fields having identical or different test chemicals comprised therein.

As further used herein, the term "test field" is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the test field having the test chemical comprised therein.

The term "capturing at least one image" as used herein is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by the user action or may automatically be initiated, e.g. once the presence of the at least one object within a field of view and/or within a predetermined sector of the field of view of the camera is automatically detected. These automatic image acquisition techniques are known e.g. in the field of automatic barcode readers, such as from automatic barcode reading apps. The capturing of the images may take place, as an example, by acquiring a stream or "life stream" of images with the camera, wherein one or more of the images, automatically or by user interaction such as pushing a button, are stored and used as the at least one first image or the at least one second image, respectively. The image acquisition may be supported by a processor of the mobile device, and the storing of the images may take place in a data storage device of the mobile device.

The at least one image of the at least one part of the optical test strip specifically may comprise an image of at least a part of the test field. Further, the image may comprise an image of other parts of the optical test strip, such as a white reference part of the test strip.

The capturing of the at least one image may comprise capturing at least one image with having the sample of the bodily fluid applied to the test strip and, further and optionally, such as before capturing the image with the sample applied to the test strip, capturing at least one image without having the sample of the body fluid applied to the test strip. The latter image specifically may be used for comparative purposes and may also be referred to as a "blank image" or "dry image". The sample application generally may take place, as an example, directly or indirectly, e.g. via at least one capillary element. The at least one image captured after sample application may typically also be referred to as the "wet image", even though the sample may have dried when the image is actually captured. The wet image typically may be taken after having waited for at least a predetermined waiting time, such as after five seconds or more, in order to allow for the detection reaction to take place. Thus, as an example, the method may comprise, between taking the at least one optional dry image and the at least one wet image, waiting for at least a predetermined minimum amount of time. This predetermined minimum amount of time specifically may be sufficient for a detection reaction to take place in the test strip. As an example, the minimum amount of waiting time may be at least 5 s.

The method, as outlined above, comprises determining the at least one analyte concentration value from color formation of the test field. Thus, the method may be an analytical measurement including a change of at least one optical property of an optical test strip, which change may be measured or determined visually by using the camera. Specifically, the analytical measurement may be or may comprise a color formation reaction in the presence of the at least one analyte to be determined. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction. The color formation may be detected by the mobile device, such as by a processor of the mobile device, and may be evaluated quantitatively, such as by deriving, from the at least one image, at least one parameter quantifying or characterizing the color formation of the test field due to the presence of the analyte in the bodily fluid. As an example, one or more of the above-mentioned color coordinates may be used. Thus, the mobile device and specifically the processor of the mobile device may be configured for determining a color change by determining a change of one or more color coordinates taking place due to the detection reaction.

The at least one analyte concentration value is determined from the color formation of the test field. For this purpose, the at least one image may be used. The analyte concentration value, as an example, may be a numerical value indicator of a result of the analytical measurement, such as indicative of the concentration of at least one analyte in the sample, such as a blood glucose concentration.

For determining the analyte concentration from the color formation of the test field, at least one correlation for transforming color formation of the test field into the analyte concentration value may be used, such as at least one correlation provided in the mobile device. The correlation, as an example, may be a correlation function such as a linear correlation, for transforming at least one item of color information derived from the at least one image into the analyte concentration. Thus, as an example, for blood glucose measurements, often, a red channel of the image and/or an R value derived from the image is transformed, by using a linear transformation, into the blood glucose concentration. Other correlations, however, are feasible. The correlation, as an example, may be provided in an electronic format, such as in a data storage and/or via at least one interface of the mobile device. The correlation, as an example and as will be outlined in further detail below, specifically may be provided in various ways, such as by providing one or more parameters defining the correlation, such as parameters defining a linear relationship between the analyte concentration value and at least one item of information derived from the at least one image. As an example, for linear correlations, and offset and a slope of the correlation may be provided. Other types of correlation are possible.

Thus, generally, as used herein, the term "correlation" as used herein is a broad term and is to be given its ordinary and customary meaning to a person of ordinary skill in the art and is not to be limited to a special or customized meaning. The term specifically may refer, without limitation, to a predetermined or determinable relationship between information derived from the at least one image, such as color information or color change information, and the at least one analytical measurement concentration value. For determining the analytical measurement result value from the at least one image, as an example, the correlation or predetermined or determinable relationship between information derived from the at least one image, such as color information or color change information, and the at least one analytical measurement result value may be used. This correlation or predetermined or determinable relationship, as an example, may be stored in a data storage device of the mobile device and/or in the processor of the mobile device. The processor, as an example, may be configured by software programming to derive at least one item of information from the at least one image, such as at least one color coordinate, and to apply the predetermined or determinable relationship to the at least one item of information. The correlation, as an example a transformation function, a transformation table or a lookup table, may be determined e.g. empirically and may, as an example, be stored in at least one data storage device of the mobile device, e.g. by the software, specifically by the app downloaded from an app store or the like. As an example for deriving the at least one item of information, the processor may be programmed in order to recognize, preferably automatically, e.g. by pattern recognition and/or other algorithms, the test field or the at least one part of the test field in the images. Thereof, the processor may be programmed for determining the at least one item of information, such as one or more color coordinates. The respective at least one item of information derived from the at least one optional blank or dry image may be used for normalizing, such as by dividing the at least one item of information derived from the wet image by the at least one item of information derived from the corresponding blank image or by subtracting the at least one item of information derived from the wet image from the at least one item of information derived from the blank image or vice a versa. Other ways of normalizing are feasible. The correlation, as an example a transformation function, a transformation table or a lookup table, may be determined e.g. empirically and may, as an example, be stored in at least one data storage device of the mobile device, e.g. by the software, specifically by the app downloaded from an app store or the like.

The correlation, as will be outlined in further detail below, generally may be determined by using empirical or semi empirical methods, such as by using a "training". The training, as an example, may comprise performing a plurality of measurements transforming the color formation into analyte concentration values and comparing the result with at least one known result and/or by using other means of preference, such as the at least one color reference card explained in further detail below. As an example, the training may also comprise the use of one or more artificial in your networks. As an example, a plurality of images may be used as input for one or more artificial neural networks for determining the analyte concentration value, by using reference information, such as from one or more color reference cards and/or from one or more reference measurements for feedback. Other means of training are also possible and generally known to the skilled person, such as by using regression methods such as linear regression, e.g. for determining parameters of the correlation. As a result of the training, the one or more parameters characterizing the correlation may be obtained.

The method may further comprise the step of displaying the analyte concentration value, such as on a display of the mobile device. Additionally or alternatively, the method may comprise storing the at least one analyte concentration value in at least one data storage device of the mobile device. Again additionally and alternatively, the method may further comprise transmitting the at least one analyte concentration value via at least one interface and/or via at least one data transmission network, such as to another computer, e.g. for further evaluation.

As further outlined above, the adjustment method, which may be a prerequisite for performing the analytical method, and which may be performed at least once before performing the analytical method, comprises, in step i), carrying out, by a plurality of users, a plurality of analyte measurements. The analyte measurements, at least partly, comprise capturing images of at least a part of an optical test strip having a test field by using the camera, thereby obtaining training data on the analyte measurements. For the analyte measurements and the performing of the analyte measurements of step i), which may also be referred to as the training analyte measurements, specifically the training analyte measurements of the adjustment method, reference may be made to the analyte measurements described above. Thus, specifically, the plurality of users, which may comprise, as an example, patients, healthy people or healthcare professionals, each may perform analyte measurements by capturing images of at least a part of an optical test strip having a test field by using the camera. Thus, as an example, the users of step i) may form a training entity or training population. Each of the users of the training population may possess at least one mobile device having at least one camera and, further, may possess at least one optical test strip having the at least one test field. The samples used in training step i) may be of the same type. Thus, as an example, each of the users of the training population may apply the same type of bodily fluid to his or her respective optical test strip, such as blood. As an example, each user may apply his or her own blood or, specifically in case of healthcare professionals, may apply a sample of a patient's blood to the optical test strip. The analyte measurements or training analyte measurements of step i) may be performed by each user applying the at least one sample of the bodily fluids to the optical test strip, capturing at least one image of at least a part of the optical test strip having the test field by using the respective camera of the individual mobile phone of the training user and, further, determining the respective analyte concentration as outlined above. Thereby, training data on the training analyte measurements performed by the plurality of users in step i) may be generated.

The term "training data" as used herein and as used, specifically, in the context of step i), is a broad term and is to be given its ordinary and customary meaning to a person of ordinary skill in the art and is not to be limited to a special or customized meaning. The term specifically may refer, without limitation, to arbitrary data relating to the training analyte measurements of step i), specifically the individual training analyte measurements performed by the users of the training population. Thus, as an example, a training data set may be generated for each training analyte measurement carried out in step i), wherein the entity of the training data sets, as an example, may form the training data. The individual training data sets of the users may be of the same type, such as by defining types of data contained in the training data sets. The training data may, specifically, comprise at least one item of information selected from the group consisting of: the at least one image of the respective training analyte measurement; at least one item of information derived from the image of the respective training analyte measurement, such as at least one item of color information; an analyte concentration value determined during the respective training analyte measurement; at least one setting parameter of the mobile device used for the respective training analyte measurement; at least one sensor parameter generated by at least one sensor of the mobile device before, during or after the training analyte measurement; at least one external sensor parameter generated by at least one external sensor before, during or after the training analyte measurement; at least one handling parameter characterizing the procedure and/or the handling of the mobile device by the user during the analyte measurement. Additionally or alternatively, one or more other parameters characterizing the respective training analyte measurement may be comprised in the training data. Further exemplary embodiments will be given below.

As further outlined above, in step ii), the training data obtained in step i) are analyzed and, thereby similarities in the training data are identified. Further, a plurality of user profiles according to the similarities in the training data are identified. The analysis may at least partially take place on the respective mobile devices of the plurality of users, such as on the individual mobile devices of the users used during the training analyte measurements. Additionally or alternatively, however, as will be outlined in further detail below, the analysis may fully or partially take place on at least one analyzing entity separate from the mobile devices of the plurality of users of step i). Thus, the at least one analyzing entity, as an example, may comprise at least one evaluation server, sometimes also referred to as an evaluation server device, which, as an example, may be a single server or a cloud server. Thus, the analysis may fully or partially take place in the cloud.

The term "analyzing" as used herein is a broad term and is to be given its ordinary and customary meaning to a person of ordinary skill in the art and is not to be limited to a special or customized meaning. The term specifically may refer, without limitation, to an arbitrary process for deriving information from input data or raw data. Thus, specifically, in the context of step ii), the analyzing of the training data comprises an arbitrary process which identifies similarities in the training data and identifies a plurality of user profiles according to the similarities. As outlined in further detail below, the analysis, for the purpose of identifying the similarities, specifically may comprise using at least one self-learning process and/or at least one process of artificial intelligence, such as at least one artificial neural network. The term "similarities" as used herein is a broad term and is to be given its ordinary and customary meaning to a person of ordinary skill in the art and is not to be limited to a special or customized meaning. The term specifically may refer, without limitation, to the property of at least two virtual or real objects and/or of at least two items of information fulfilling one or more similarity conditions and/or one or more similarity criteria. Therein, similarities may be quantified by using one or more similarity functions and/or using similarity learning techniques known to the skilled person in the field of supervised machine learning in artificial intelligence. Each set of training data comprises a variable, wherein the similarities of two sets of training data are defined by the values of the respective variables being in a specific range or being spaced apart by no more than a given threshold. Other similarity criteria, however, are feasible in addition. The result of the identification of similarities, as an example, may comprise information on which of the training data sets are similar with regard to which variables and/or parameters contained therein. Other similarities, however, may also be detected.

The term "user profile" as used herein is a broad term and is to be given its ordinary and customary meaning to a person of ordinary skill in the art and is not to be limited to a special or customized meaning. The term specifically may refer, without limitation, to at least one item of information on the user. The user profile comprises at least one item of information relating to one or more of: a physical state of the user; a habit of the user, such as a mode of the user's acting and/or the user's response to specific situations; a degree of experience of the user, such as a degree of experience relating to the handling of the mobile device and/or the analytical method; a user's personal situation, such as the mobility of the user. The user profile specifically may relate to a specific category of users, such that the terms "category" and "profile" may also be used as synonyms. By identifying the plurality of user profiles according to the similarities in the training data, thus, the users may be categorized into at least two different categories, wherein the users in one category has the same or at least similar user profiles. New users, thus, may be analyzed, as will be outlined in further detail below with respect to the description of the analytical method, and may either be assigned to an existing category in accordance with their respective user profile or, alternatively, may be assigned to a new category, in case the new users do not fit into any existing category. Thus, the training described above may also be performed repeatedly, iteratively or continuously, in order to identify new user profiles and/or new user categories as soon as they occur. The user profiles, as indicated above, may contain a plurality of items of information, such as specific variables. The different categories or user profiles may, as an example, be defined by different values of different value ranges of these variables. Each user may be assigned to exactly one user profile or category of users. Alternatively, however, the user may also be assigned to more than one user profile or user category, such as in case overlapping user profiles are categories are chosen. As an example, user categories "experienced" and "inexperienced" may be defined, such as in accordance with a specific set of variables indicating specific skills or indicating specific reactions or reaction times of the users. A user assigned to the category "experienced", however, may also be assigned to one or more other categories. Thus, one or more profiles may be generated for specific types of models of the mobile device. Additionally or alternatively, one or more profiles may be generated or identified, indicating a specific physical state. Other examples are feasible.

As further outlined above, in step iii), profile-specific measurement setup adjustments are provided for at least one of the user profiles. The setup adjustments, as an example, may be provided in an electronic format, such as in at least one data storage device, e.g. a data storage device of a server and/or a cloud server, and/or via at least one interface, such as a web interface. The term "profile-specific measurement setup adjustments" as used herein is a broad term and is to be given its ordinary and customary meaning to a person of ordinary skill in the art and is not to be limited to a special or customized meaning. The term specifically may refer, without limitation, to at least one specific setting for carrying out the at least one analyte measurement by a user, the user belonging to a specific user profile. The setting, specifically, may refer to at least one of a variable, a device of the mobile device or instructions for performing the analyte measurement, such as instructions displayed on a display of the mobile device. The term "adjustments", as used therein, may refer to maintaining a specific setting or changing a specific setting. As an example, the profile-specific measurement setup adjustments specific to for a specific user profile may comprise one or more of instructions, commands, variables, values or program code to be provided to the mobile device of the user belonging to the user profile, in order to have the mobile device perform a specific process for the analyte measurement, such as a specific analyte measurement or process of analyte measurement suited for users of the user profile. As an example, in case the user profile is a profile for users having a specific illness, such as an illness leading to a frequent tremor, the profile-specific measurement setup adjustments, as will be outlined in further detail below, may comprise increased tolerance for motion blur, such as motion blur of the images used for analysis, as compared to e.g. users of profiles not indicating a frequent tremor. Further, in case the user profile is a profile for an experienced user, user instructions, e.g. instructions displayed on a display of the mobile device, may be less extensive as compared to user instructions displayed for users of a profile indicating a lower experience. Other options are feasible, and further exemplary embodiments will be given below.

The profile-specific measurement setup adjustments specifically at least partially may refer to at least one of:
- a handling procedure of the analyte measurement;
- a hardware setup of the mobile phone;
- a software setup of the mobile phone;
- instructions given by the mobile phone to the user;
- a degree of reliability of measurement result of the analyte measurement;
- a tolerance range for admissible parameters when performing the analyte measurement;
- a timing sequence for performing the analyte measurement;
- a failsafe algorithm for performing the analyte measurement;
- an enhanced analyte measurement accuracy.

Other options are feasible additionally or alternatively.

As outlined above, step i) comprises obtaining or generating training data. The training data comprises at least one of:
- color information derived from the images;
- information derived from at least one color reference card visible in the images;
- analyte measurement values derived from the images;
- sensor data obtained by using at least one sensor of the users' mobile phones selected from the group consisting of: an angle sensor; a light sensor; a motion sensor; an acceleration sensor; a gyroscopic sensor; a magnetic sensor, specifically a Hall sensor; a GPS sensor; a pressure sensor, specifically a barometer; a temperature sensor; a biometric sensor, specifically one or more of a fingerprint sensor and/or an iris scan sensor;
- optionally sensor data obtained by using at least one external sensor;
- setup information relating to a setup of the users' mobile devices for carrying out the analyte measurements, specifically setup information relating to at least one of an illumination, a sensitivity, an exposure time, a Gamma correction;
- health information relating to the users, specifically health information obtained from health Apps running on the users' mobile devices.

Thus, generally, the training data may also comprise information in addition to information derived from the images, such as analytical information. As an example, user-specific preferences or settings of the mobile devices used for generating the training data may be considered, as well as external or internal sensor data. Further, the training data may also consider information provided by the users, either actively by user input or passively by considering data contained in the mobile phones, such as health applications running on the mobile device. Further, data on relating to the mobile device itself may be considered, such as on the type of the mobile device and/or on settings of the mobile device. Further, environmental data may be considered, such as data on the typical situation of measurement, e.g. user preferences regarding lighting conditions or the like.

Step ii) specifically may comprise using at least one self-learning algorithm, more specifically at least one artificial intelligence. Thus, step ii) may comprise using one or more of a trained model and an artificial neural network, such as a convolutional neural network. As an example, generic artificial neural networks may be adapted for the purposes of the analysis of the training data, such as TensorFlow^{®}. Other artificial neural networks, however, may also be used.

The adjustment method may further comprise transmitting the training data obtained in step i) from the respective users' mobile devices to at least one evaluation server device. The term "evaluation server device", also referred to as an "evaluation server", as used herein is a broad term and is to be given its ordinary and customary meaning to a person of ordinary skill in the art and is not to be limited to a special or customized meaning. The term specifically may refer, without limitation, to a device which is capable of performing one or more evaluation operations. Specifically, the evaluation device may comprise at least one processor, which may be programmed, by appropriate software, for performing one or more evaluation operations. The at the least one evaluation server device specifically may be at least one cloud-based evaluation server device, also referred to as a cloud server. Generally, at least step ii) and optionally also step iii) as well as, optionally, one or more further steps may be performed by the evaluation server device. Thus, specifically, the analysis of the training data and optionally also the providing of the profile-specific measurement setup adjustments may be performed by the evaluation server device. Alternatively, however, step iii), i.e. providing the profile-specific measurement setup adjustments for at least one of the user profiles, may also fully or partially be performed by a separate entity, such as by a server device being separate from the evaluation server device and/or by the individual user's mobile device used for the analytical method and/or by one more of the mobile devices of the plurality of users involved in the adjustment method. Other options are feasible.

The similarities identified in step ii) at least partially refer to lighting conditions when performing step i). The profile-specific measurement setup adjustments at least partially refer to camera adjustments when carrying out step i), specifically to camera adjustments referring to at least one of: a sensitivity of the camera; an exposure time. Thereby, the profile specific measurement setup adjustments may take into account similarities of the users of a specific category or profile with respect to the lighting conditions during analyte measurements. As an example, users typically performing analyte measurements under artificial light may be assigned to a specific profile, taking into account e.g. the specific spectral properties of the artificial light.

The similarities identified in step ii), additionally, at least partially refer to a users' tremor when performing step i). Thus, users having a significant tremor may be assigned to a specific profile. The profile-specific measurement setup adjustments, in this case, at least partially refer to one or more of: an admissible range of movement of the mobile phone when performing step i); image blur of the images captured in step i). Thus, by identifying tremor, user-specific adjustments of the mobile device taking into account the uses tremor may be performed.

In a further aspect of the present invention, an analytical method of determining a concentration of an analyte in a bodily fluid is disclosed. The analytical method comprises using a mobile device having a camera. The method comprises capturing at least one image of at least a part of an optical test strip having a test field by using the camera. The method further comprises determining at least one analyte concentration value from color formation of the test field. For possible definitions, reference may be made to the description of the adjustment method above. The analytical method further comprises the following steps which specifically may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed. The analytical method comprises the following steps:
a) performing the adjustment method according to the present invention, such as according to any one of the embodiments described above and/or according to any one of the embodiments disclosed in further detail below;
b) carrying out, by at least one individual user, a plurality of analyte measurements, wherein the analyte measurements, at least partly, comprise capturing images of at least a part of an optical test strip having a test field by using the camera, thereby obtaining user-specific training data on the analyte measurements;
c) analyzing the user-specific training data obtained in step b) and assigning the individual user to at least one individual user profile of the user profiles; and
d) providing user profile-specific measurement setup adjustments for the individual user profile.

The analytical method, thus, distinguishes between the plurality of users, also referred to as test users, carrying out step i) of the adjustment method described above, and the individual user carrying out step b) of the analytical method. The individual user may be part of the entity of test users or may be distinct from the test users. In general, however, it is possible to integrate the individual user, at least after carrying out step b) and preferably also step c), into the entity of test users. Consequently, steps b) and optionally c) may also be part of steps i) and optionally ii), even though, in the steps of the analytical method, the individual user and the analytical method performed by the individual user is in focus, in order to provide profile specific measurement setup adjustments for the individual user.

For most of the definitions and options of the analytical method, reference may be made to the description of the adjustment methods described above. In step b), the individual user specifically may perform the plurality of analyte measurements, which also may be referred to as individual training measurements, by using his or her mobile device. Further, the individual user may use samples of his or her own bodily fluid for the purpose of the individual training measurements of step b). Alternatively, however, one or more healthcare persons may be involved in the individual training measurements.

For details of the individual training measurements of step b), specifically for details on the capturing of the images, the optical test strips and further details, reference may be made to the description of the adjustment method above. The carrying out of the training measurements in step b) may also involve determining an analyte concentration value for these training measurements or at least for one or more of these training measurements. For the purpose of the determination of the analyte concentration for the training measurements, again, a correlation between at least one item of information derived from the images and the analyte concentration value may be used. This correlation, as an example, may be or may comprise a preliminary correlation, which, after applying the profile specific measurement setup adjustments for the individual user profile in step d), may be refined.

As will be outlined in further detail below, the analyzing of the user specific training data of the individual user in step c) may fully or partially be performed, specifically, by the at least one evaluation server device. Additionally or alternatively, however, at least a part of the analysis may also take place on the individual user's mobile device, such as on a processor of the individual user's mobile device.

As will also be outlined in further detail below, the providing of the user specific measurement setup adjustments for the individual user profile specifically may also, optionally, take place by providing the adjustments for the individual user from the evaluation server device to the individual user's mobile device. Alternatively, however, providing the user specific measurement setup adjustments may also fully or partially be performed by a separate entity to the individual user's mobile device. Other options, however, are feasible.

The analytical method may further comprise, specifically before step b), the following step:
e) assigning the individual user to a predefined starting profile.

This predefined starting profile, specifically, may comprise the at least one preliminary correlation mentioned above. Additionally or alternatively, the predefined starting profile, which may be used as a default starting profile, may comprise predefined settings which may represent an average user, such as a user having one or more of: an average knowledge; average capabilities of handling the analyte measurement; an average correlation between the at least one item of information derived from the image and the analyte concentration value; average handling conditions such as average lighting conditions; average physical conditions. This predefined starting profile, specifically after performing steps b) and optionally one or both of steps c) and d), may be refined and may fully or partially be replaced by the user profile-specific measurement setup adjustments for the individual user profile to which the individual user is assigned.

The analytical method may further comprise transmitting the user-specific training data obtained in step b) from the individual user's mobile device to at least one evaluation server device. As outlined above, the evaluation server device may fully or partially be identical to the evaluation server device used for training steps ii) to iii). Alternatively, however, the evaluation server device may also be fully or partially separate from this training evaluation server device. Generally, the evaluation server device may be a cloud-based evaluation server device. Other options, such as non-cloud-based evaluation server devices, are also feasible. Generally, at least steps c) and d) may, at least partially, be performed by the evaluation server device. The user profile-specific measurement setup adjustments for the individual user profile specifically may be transmitted from the evaluation server device to the individual user's mobile device.

The analytical method may further comprise the following steps:
f) carrying out, by the individual user using the individual user's mobile device, at least one analyte measurement, wherein the analyte measurement, at least partly, comprise capturing at least one image of at least a part of an optical test strip having a test field by using the camera, and evaluating the at least one image for deriving at least one measurement value of the concentration of the analyte in the bodily fluid, wherein the analyte measurement is performed by using the user profile-specific measurement setup adjustments for the individual user.

Thus, in step f), the individual user may actually perform the analyte measurement as described above, using his or her mobile device and his or her at least one optical test strip, with the user profile-specific measurement setup adjustments for the individual user. Step f), thus, may be an application of the result of training steps i) to iii). Consequently, steps i) to iii) and steps b) and c) may be performed at least once before performing step f).

The user-specific training data specifically may comprise training data as described above in the context of the training data of the adjustment method. Thus, for the possible types or content of the user-specific training data, reference may be made to the training data of step i), wherein the user of the plurality of users of step i) may be replaced by the individual user.

Step c) specifically may comprise using at least one self-learning algorithm. More specifically, step c) may comprise using at least one artificial intelligence, more specifically an artificial neural network, such as a convolutional neural network. Specifically, the artificial network may be used for detecting similarities between the user-specific training data and the training data of the users of one of the user profiles identified in step ii). Therein, step c) may be comparable to the analyzing step ii), since artificial neural networks may generally be used for detecting similarities. For possible definitions or details, such as on the nature of the similarities, reference may be made to the description of step ii) above. In accordance with the similarities, the individual user may be assigned to at least one of the user profiles, which thereby is defined to be the at least one individual user profile. As outlined above, the individual user may be assigned to one individual user profile or to a plurality of individuals user profiles.

In a further aspect of the present invention, an adjustment system for performing the adjustment method according to the present invention, such as according to any one of the embodiments described above and/or according to any one of the embodiments described in further detail below, is disclosed. The term "adjustment system" as used herein is a broad term and is to be given its ordinary and customary meaning to a person of ordinary skill in the art and is not to be limited to a special or customized meaning. The term specifically may refer, without limitation, to a system having one or more of the system components, the system being configured for performing the method. Thus, as an example, the system may comprise one or more computers, such as one or more computers each having at least one processor, wherein the computers are programmed to perform the method. For most of the functions and components of the adjustment system, reference may be made to the description of the adjustment method given above. The adjustment system comprises:
I) a receiving device for receiving training data on analytical measurements, the training data being obtained by a plurality of users carrying out a plurality of analyte measurements, wherein the analyte measurements, at least partly, comprise capturing images of at least a part of an optical test strip having a test field by using the camera, thereby obtaining training data on the analyte measurements;
II) an evaluation server device configured for performing step ii) of the adjustment method and optionally ; and
III) a transmission device for transmitting profile-specific measurement setup adjustments provided in step ii).

The term "receiving device" as used herein is a broad term and is to be given its ordinary and customary meaning to a person of ordinary skill in the art and is not to be limited to a special or customized meaning. The term specifically may refer, without limitation, to an arbitrary device configured for receiving data, such as a wireless or wire-bound interface for receiving data. As an example, the receiving device for receiving the training data may comprise a web interface. Similarly, the term "transmission device" as used herein is a broad term and is to be given its ordinary and customary meaning to a person of ordinary skill in the art and is not to be limited to a special or customized meaning. The term specifically may refer, without limitation, to an arbitrary device configured for transmitting data, such as a wireless or wire-bound interface for transmitting data, specifically a web interface. The receiving device and the transmission device may be separate devices. However, the receiving device and the transmission device may also be fully or partially identical.

For definitions of the term "evaluation server device", also simply referred to as an "evaluation server", reference may be made to the description of the methods above. Specifically, the evaluation server may be or may comprise at least one cloud-based server, also referred to as a cloud-based evaluation server device. Other embodiments, however, are feasible.

In a further aspect of the present invention, a mobile device is disclosed, specifically configured for use by the individual user as described above. The mobile device comprises at least one camera. The mobile device further is configured, such as by programming of at least one processor of the mobile device, for:
- performing step b) of the analytical method according to the present invention, such as according to any one of the embodiments described above and/or according to any one of the embodiments described in further detail below;
- receiving the user profile-specific measurement setup adjustments for the individual user profile provided in step d).

Thus, the mobile device may be configured for performing all of the method steps or for performing the method steps carried out by the individual user. Specifically, the mobile device may be configured for performing step b) of the method. Optionally, the mobile device may be configured for transmitting the user-specific training data obtained thereby to the evaluation server device. The mobile device may further be configured for receiving the profile specific measurement setup adjustments for the individual user profile, such as for receiving these setup adjustments from the evaluation server device.

The mobile device may further be configured use of these profile specific measurement setup adjustments for one or more subsequent analytical measurements performed by the individual user. Thus, the mobile device may further be configured for performing step f) as outlined above, by using the user profile-specific measurement setup adjustments for the individual user.

The mobile device specifically, as outlined above, may comprise at least one processor. The term "processor" as generally used in this description is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like. The processor specifically may be configured, such as by software programming, for performing one or more evaluation operations.

The mobile device may be configured for performing further steps. Thus, specifically, the mobile device may be configured for providing evaluation support performing at least one evaluation step for the analyte measurement, as outlined above. The evaluation may also be supported by artificial intelligence running on the mobile device, such as by at least one artificial neural network. Thus, as an example, at least one artificial neural network and/or other artificial intelligence may be used for determining or detecting extraordinary measurement values or outliers. Other embodiments are feasible.

In a further aspect of the present invention, a computer program is disclosed, comprising instructions which, when the program is executed by a mobile device having a camera, specifically by a processor of the mobile device, more specifically by the mobile device according to the present invention such as according to any one of the embodiments disclosed above and/or according to any one of the embodiments disclosed in further detail below, cause the mobile device to carry out at least method steps b) and f) of the analytical method as described above. Similarly, a computer-readable storage medium is disclosed, comprising instructions which, when executed by a mobile device having a camera, specifically by a processor of the mobile device, more specifically by the mobile device according to the present invention such as according to any one of the embodiments disclosed above and/or according to any one of the embodiments disclosed in further detail below, cause the mobile device to carry out at least method steps b) and f) of the analytical method as described above. As used herein, the term "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

In a further aspect, a computer program is disclosed, comprising instructions which, when the program is executed by the adjustment system according to the present invention, such as according to any one of the embodiments disclosed above and/or according to any one of the embodiments disclosed in further detail below, cause the adjustment system to carry out the adjustment method according to the present invention, such as according to any one of the embodiments disclosed above and/or according to any one of the embodiments disclosed in further detail below. Similarly, a computer-readable storage medium is disclosed, comprising instructions which, when the program is executed by the adjustment system according to the present invention, such as according to any one of the embodiments disclosed above and/or according to any one of the embodiments disclosed in further detail below, cause the adjustment system to carry out the adjustment method according to the present invention, such as according to any one of the embodiments disclosed above and/or according to any one of the embodiments disclosed in further detail below.

The methods, systems and devices according to the present invention provide a large number of advantages over known methods and devices of similar kind. Specifically, account may be taken of the vast amount of combined influencing factors and the ongoing release of new smartphones using improved and/or new technology, without the necessity for an up-front generation of training data in dedicated studies. The users may generate the training data at least partially themselves and may contribute, such as repeatedly or constantly, to the improvement of the analytical measurements. Thereby, user-specific adjustments may be performed to the mobile devices, and user-specific profiles may be generated and used by the individual user, taking into account individual habits, individual capabilities, individual health situations, individual living circumstances and the like.

Further, extensive market research, which typically is expensive and in many cases not continuously given, may be avoided. Optimization and failsafe actions may be taken immediately and continuously, and by providing the user-specific profiles complaints by individual users may be avoided.

The training data may be collected via the Internet, such as using TCP/IP or other protocols, such as Internet Printing Protocol (IPP). User profiles and profile-specific measurement setup adjustments may be derived, such as by deriving optimized or at least improved measurement setups. Thus, measurement setups referring, as an example, to one or more of measurement sequences, measurement algorithm failsafes or other customer specific properties may be derived. Specifically, cloud processing may be used in order to obtain more detailed user handling data and/or individual health status data and make the data available for providing the measurement setup adjustments.

The training data may make use of one or more sensors, internal and/or external to the mobile phones used in one or more of steps i) and/or b). Therein, as an example, a variety of sensors may be used, such as one or more a gyroscope, an accelerometer, a proximity sensor, an ambient light sensor, a GPS sensor, a barometer, a Hall-sensor, a thermometer or a biometric sensor such as fingerprint sensors and/or an iris sensor. Sensor data from one or more of these internal or external sensors can also be added to the training data generated in one or more of steps i) and/or b) and can be used to derive user-specific profiles such as handling patterns.

Additionally or alternatively, health data may be raised and added to the training data in one or both of steps i) and/or b), such as health data like heart rate and the like. As an example, the training data may consider data provided via one or more health Apps such as Samsung health or Apple health. These health App data may provide additional customer and/or customer group specific information. The invention, thus, allows for combining training data comprising data obtained in one or both of steps i) or b) with other data obtained by other means than analyte measurements. Thus, analyte measurement training data in combination with other data such as user-related data can be used to derive profile-specific measurement setup adjustments such as plausibility failsafe checks or other adjustments. Further, by providing the user profiles, a basic model for the user groups, e.g. for each user group, may be generated, such as by indicating that a user is a new user of diabetes type 2. Profile-specific measurement setup adjustments may be provided to the individual users, by learning from previous training, such as by performing a plurality of analyte measurements such as blood glucose measurements.

The profile-specific measurement setup adjustments may, as an example, take into account the daily routine of the user. Thus, as an example, for blood glucose measurements, the typical user profile may be based on the assumption of a routine of taking breakfast, lunch and dinner. Specific adaption, however, may be implemented for shift workers or other meal adjustments.

Further, as outlined above, the profile-specific measurement setup adjustments may take into account the personal skills or experience of the user, of the users in step i) and/or of the individual user of step b). As an example, an advanced control of the measurement sequence may be implemented into the adjustments. For this purpose, the handling of the analyte measurement using the mobile phone by the user may be analyzed, in order to identify handling optimization potential and/or failsafe options. As an example, acceleration sensor data and/or gyroscope data may be implemented into the training data generated in step i) and/or into the user-specific training data of step b). As an example, a timing may take place when using an App running on the mobile device. Therein, timing tracking data or screen tracking data may be generated, such as data referring to the question how long each screen was visible and/or how fast the next step was initiated by the user. Thereby, user-specific handling procedures may be identified, such as how long each screen was watched by the user. Further, motion data may be used for identifying when and how the mobile device was moved, in order to defuse a level of experience. Further, profiles may be classified into good profiles and bad profiles or into other categories, depending on evaluation criteria. Further, additionally or alternatively, fitness Apps running on the mobile devices could be used for detecting which kind of sport is being performed, in order to train a standard measurement sequence and variants thereof.

A further example of user-specific measurement setup adjustments are adjustments allowing for specific misalignments and/or blur in the images. Thus, typically, the image is captured in one or both of steps i) and b) require a certain quality which might be checked automatically by software. However, it might be advisable to allow for a certain amount of motion blur for certain groups of users, such as for people having a tremor. Again, acceleration sensor data and/or gyroscope data may be used for evaluating the physical state of the user, in one or both of steps ii) or c). Thus, as an example, at least one group of users having a tremor may be identified, and the profile-specific measurement setup adjustments for this group of users might imply lowering the quality requirements of the images. As a standard profile, the recorded image may be analyzed to detect motion blur and reject images having a motion blur above a specific tolerance. Thereby, for standard profiles, a correct orientation of the camera to the test strip may be ensured and images in which the mobile phone is still in motion when capturing the image may be rejected. In order, however, to allow for a user having a strong tremor to take part in the measurements, the level of tolerance may be lowered for these users. Typically, for measuring color, and, thus, for deriving information for obtaining the analyte measurement data, a sharp image is not a strict prerequisite. The analysis in one or both of steps ii) and c) may also distinguish between motion blur resulting from larger movements of the mobile device in space and blur resulting from semi-periodic movement due to tremor.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of an adjustment system and of a mobile device; and
- Figure 2: shows a flow chart of an embodiment of an adjustment method of adjusting a measurement setup for an analytical method of determining a concentration of an analyte in a bodily fluid; and
- Figures 3and 4: show flow charts of different embodiments of an analytical method of determining a concentration of an analyte in a bodily fluid.

### Detailed description of the embodiments

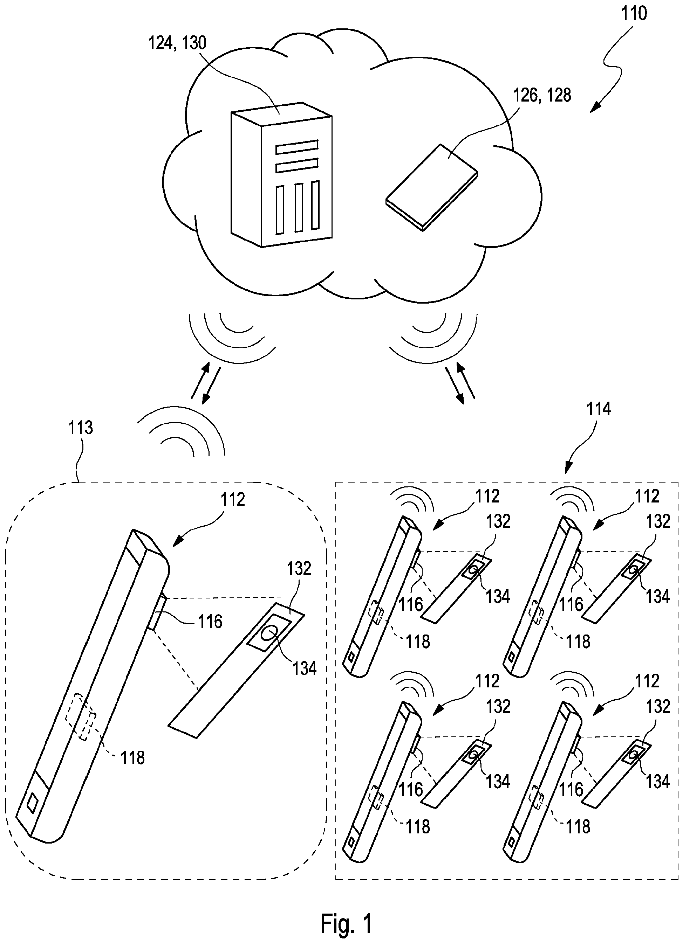

In Figure 1, embodiments of an adjustment system 110 and of a mobile device 112 according to the present invention are shown in a schematic view. Further, a plurality of users 114 is shown symbolically, each with their own mobile device 112.

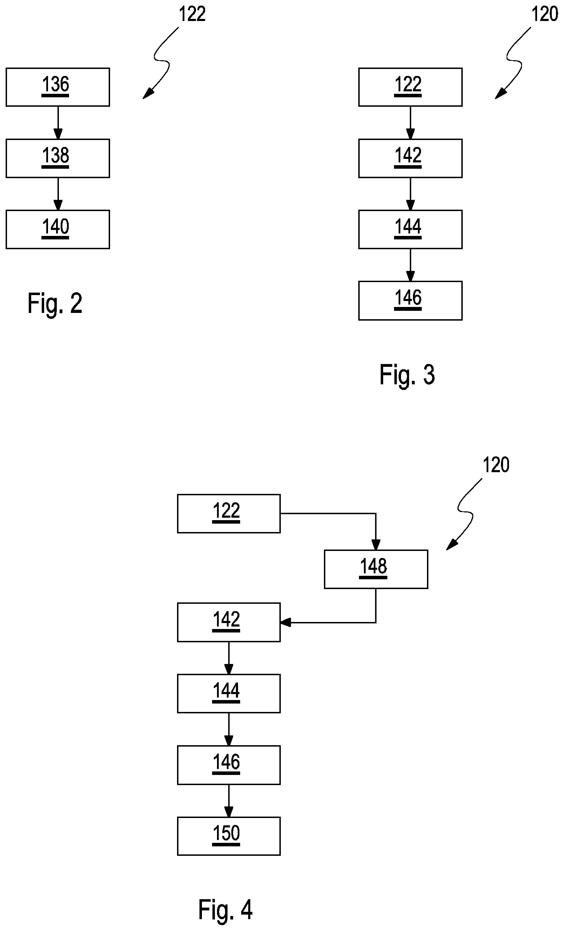

The mobile device 112 has at least one camera 116 and at least one processor 118. Further, the mobile device 112 is configured for performing at least partially an analytical method 120 of determining a concentration of an analyte in a bodily fluid and for performing at least partially an adjustment method 122 of adjusting the measurement setup for the analytical method 120. The analytical method 120 will be described in detail in Figure 3 and 4; the adjustment method 122 will be described in Figure 2.

The adjustment system 110 comprises an evaluation server device 124 and a receiving device 126 as well as a transmission device 128. The receiving device 126 and the transmission device 128 may be fully or partially identical and may also fully partially be implemented into the evaluation server device 124. The evaluation server device 124 may fully or partially be embodied as a cloud-based evaluation server device 130.

The evaluation server device 124 may be configured to communicate with both the mobile device 112 of an individual user 113 and the mobile devices 112 of the plurality of users 114. In particular, at least one item of information may be transmitted to the evaluation server device 124 and at least one item of information may be transmitted by the evaluation server device 124 to one or both of the mobile device 112 of the individual user 113 and/or the mobile devices 112 of the plurality of users 114, as illustrated in Figure 1 by the two arrows pointing in opposite directions.

The processor 118 of each the mobile device 112 may be configured to capture and process an image of an optical test strip 132 having a test field 134, taken by the camera 116 of the mobile device 112.

A flow chart of an exemplary embodiment of an adjustment method 122 is shown in Figure 2. The adjustment method 122 is adapted for adjusting a measurement setup for an analytical method 120 of determining a concentration of an analyte in a bodily fluid. The analytical method 120 will be described in detail in Figures 3 and 4.

The adjustment method 122 comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The adjustment method 122 may comprise additional method steps that are not listed. The method steps of the adjustment method 122 are the following:
i) (denoted with reference number 136) carrying out, by the plurality of users 114, a plurality of analyte measurements, wherein the analyte measurements, at least partly, comprise capturing images of at least a part of the optical test strip 132 having the test field 134 by using the camera 116, thereby obtaining training data on the analyte measurements;
ii) (denoted with reference number 138) analyzing the training data obtained in step i) 136, thereby identifying similarities in the training data and identifying a plurality of user profiles according to the similarities in the training data; and
iii) (denoted with reference number 140) providing profile-specific measurement setup adjustments for at least one of the user profiles.

In particular, the training data comprises: color information derived from the image, information from at least one color reference card, analyte measurement values derived from the image, sensor data by using at least one sensor of the users' mobile device 112 or, optionally, obtained by at least on external sensor, setup information relating to a setup of the users' mobile device 112 or health information about the user, e.g. obtained from health Apps running on the users' mobile device 112.

The training data obtained in step i) 136 may be transmitted from the mobile devices 112 of the plurality of users 114 to the at least one evaluation server device 124, specifically to the cloud-based evaluation server device 130. Step ii) 138 and optionally also step iii) 140 may be performed on the evaluation server device 124. Step ii) 138 of the adjustment method 122 may comprise using at least one self-learning algorithm, specifically by using at least one artificial intelligence, more specifically by using one or more of a trained model and an artificial neural network and more specifically by using a convolutional neural network. By identifying the plurality of user profiles according to the similarities in the training data, thus, the users of the plurality of users 114 may be categorized into a plurality of categories, specifically into at least two different categories, wherein the users assigned to a specific category may have, at least with respect to specific properties, the same or at least similar user profiles. The similarities obtained in step ii) 138 from analyzing the training data refer, at least partially, to the users' tremor when performing step i) 136.

In step iii) 140, profile-specific measurement setup adjustments are provided for at least one of the user profiles. The profile-specific measurement setup adjustments at least partially refer to a handling procedure of the analyte measurement, a hardware and/or software setup of the mobile device 112, instructions given by the mobile device 112 to the user, a degree of reliability of measurements result of the analyte measurement, a tolerance level for admissible parameters when performing the analyte measurement, a timing sequence or a failsafe algorithm for performing the analyte measurement and/or an enhanced analyte measurement accuracy. In particular, the profile-specific measurement setup adjustments may comprise increased tolerance for motion blur as compared to e.g. users of profiles not indicating a frequent tremor. The profile-specific measurement setup adjustments at least partially refer to one or more of an admissible range of movement of the mobile device 112 when performing step i) 136 and image blur of the images captured in step i) 136.

As another example, user categories "experienced" and "unexperienced" may be defined in step ii) 138, such as in accordance with a specific set of variables indicating specific skills or specific reactions or reaction times of the user. In case the user profile is a profile for an experienced user, user instructions, e.g. instructions displayed on a display of the mobile device 112, may be less extensive as compared to user instructions displayed for users of a profile indicating a lower experience.

Further, the similarities identified in step ii) 138 at least partially refer to lighting conditions when performing step i) 136. Therefore, profile-specific measurement setup adjustments at least partially refer to camera adjustments when carrying out step i) 136, specifically to camera 116 adjustments referring to at least one of a sensitivity of the camera 116 and an exposure time.

An example of an analytical method 120 of determining a concentration of an analyte in a bodily fluid is shown as a flow chart in Figure 3. The analytical method 120 comprises using, e.g. by individual user 113, a mobile device 112 having a camera 116. The method comprises capturing, e.g. by the individual user 113 using mobile device 112 with camera 116, at least one image of at least a part of an optical test strip 132 having a test field 134 by using the camera 116. From color formation of the test field 134, at least one analyte concentration value is determined. The analytical method 120 further comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The analytical method 120 may comprise additional steps that are not listed. The analytical method 120 comprises the following method steps:
a) performing the adjustment method 122 according to any one of the claims 1-9;
b) (denoted by reference number 142) carrying out, by at least one individual user, a plurality of analyte measurements, wherein the analyte measurements, at least partly, comprise capturing images of at least a part of an optical test strip 132 having a test field 134 by using the camera 116, thereby obtaining user-specific training data of the individual user 113 on the analyte measurements;
c) (denoted by reference number 144) analyzing the user-specific training data obtained in step b) 142 and assigning the individual user 113 to at least one individual user profile of the user profiles; and
d) (denoted by reference number 146) providing user profile-specific measurement setup adjustments for the individual user profile.

Further, as exemplarily illustrated by Figure 4, the analytical method 120 may comprise an additional step e) 148, in which the individual user is assigned to a predefined starting profile. Another additional step f) 150 may comprise carrying out at least one analyte measurement by the individual user 113 using the individual user's mobile device 112.

Further, the analyte measurement, at least partially, comprises capturing at least one image of at least a part of an optical test strip 132 having a test field 134 by using the camera 116. At least one image is evaluated and at least one measurement value of the concentration of the analyte of the bodily fluid is derived. The analyte measurement may include a change of at least one optical property of the optical test strip 132, which change may be measured or visually determined by the camera 116. Specifically, the measurement may be or may comprise a color formation reaction in the presence of the at least one analyte to be determined. The color formation may be detected by the mobile device 112, such as by the processor 118 of the mobile device 112, and may be evaluated quantitatively, such as by deriving, from the at least one image, at least one parameter quantifying or characterizing the color formation of the test field 134 due to the presence of the analyte in the bodily fluid. For determining the analyte concentration from the color formation of the test field 134, at least one correlation, such as a correlation function, for transforming color formation of the test field 134 into the analyte concentration value may be used, such as at least one correlation provided in the mobile device 112. The analyte measurement is performed by using the user profile-specific measurement setup adjustments for the individual user. The corresponding flow chart is shown in Figure 4.

### List of reference numbers

- 110: adjustment system
- 112: mobile device
- 113: individual user
- 114: plurality of users
- 116: camera
- 118: processor
- 120: analytical method
- 122: adjustment method
- 124: evaluation server device
- 126: receiving device
- 128: transmission device
- 130: cloud-based evaluation server device
- 132: optical test strip
- 134: test field
- 136: step i)
- 138: step ii)
- 140: step iii)
- 142: step b)
- 144: step c)
- 146: step d)
- 148: step e)
- 150: step f)

## Claims

1. An adjustment method (122) of adjusting a measurement setup for an analytical method (120) of determining a concentration of an analyte in a bodily fluid, the analytical method (120) comprising using a mobile device (112) having a camera (116), the analytical method (120) further comprising capturing at least one image of at least a part of an optical test strip (132) having a test field (134) by using the camera (116), and wherein the analytical method (120) further comprises determining at least one analyte concentration value from color formation of the test field (134), wherein the adjustment method (122) comprises:
i) carrying out, by a plurality of users (114), a plurality of analyte measurements, wherein the analyte measurements, at least partly, comprise capturing images of at least a part of an optical test strip (132) having a test field (134) by using the camera (116),
**characterized by**:
thereby obtaining training data on the analyte measurements,
wherein the training data comprise at least one of:
- color information derived from the images;
- information derived from at least one color reference card visible in the images;
- analyte measurement values derived from the images;
- sensor data obtained by using at least one sensor of the users' mobile devices (112) selected from the group consisting of: an angle sensor; a light sensor; a motion sensor; an acceleration sensor; a gyroscopic sensor; a magnetic sensor; a GPS sensor; a pressure sensor; a temperature sensor; a biometric sensor;
- setup information relating to a setup of the users' mobile devices (112) for carrying out the analyte measurements;
- health information relating to the users;
ii) analyzing the training data obtained in step i) (136), thereby identifying similarities in the training data and identifying a plurality of user profiles according to the similarities in the training data, wherein each set of training data comprises a variable, wherein the similarities of two sets of training data are defined by the values of the respective variables being in a specific range or being spaced apart by no more than a given threshold, wherein the similarities identified in step ii) (138) at least partially refer to at least one of the following:
- lighting conditions when performing step i);
- to the users' tremor when performing step i);
wherein the user profile comprises at least one item of information on the user relating to one or more of: a physical state of the user; a habit of the user; a degree of experience of the user; a user's personal situation; and
iii) providing profile-specific measurement setup adjustments for at least one of the user profiles, wherein the profile-specific measurement setup adjustments refer to at least one specific setting for carrying out the at least one analyte measurement by the user, the user belonging to a specific user profile, wherein the profile-specific measurement setup adjustments at least partially refer to one or more of:
- camera adjustments when carrying out step i) in case the similarities identified in step ii) at least partially refer to lighting conditions when performing step i);
- an admissible range of movement of the mobile device (112) when performing step i) in case the similarities identified in step ii) at least partially refer to a users' tremor when performing step i);
- image blur of the images captured in step i) in case the similarities identified in step ii) at least partially refer to a users' tremor when performing step i).

2. The adjustment method (122) according to the preceding claim, wherein step ii) (138) comprises using at least one self-learning algorithm.

3. The adjustment method (122) according to any one of the preceding claims, wherein the adjustment method (122) comprises transmitting the training data obtained in step i) (136) from the users' mobile devices (112) to at least one evaluation server device (124), wherein at least step ii) (138) is performed by the evaluation server device (124).

4. The adjustment method (122) according to any one of the preceding claims, wherein the profile-specific measurement setup adjustments at least partially refer to at least one of:
- a handling procedure of the analyte measurement;
- a hardware setup of the mobile device (112);
- a software setup of the mobile device (112);
- instructions given by the mobile device (112) to the user;
- a degree of reliability of measurement result of the analyte measurement;
- a tolerance range for admissible parameters when performing the analyte measurement;
- a timing sequence for performing the analyte measurement;
- a failsafe algorithm for performing the analyte measurement;
- an enhanced analyte measurement accuracy.

5. An analytical method (120) of determining a concentration of an analyte in a bodily fluid, the method comprising using a mobile device (112) having a camera (116), wherein the method comprises capturing at least one image of at least a part of an optical test strip (132) having a test field (134) by using the camera (116), and wherein the method further comprises determining at least one analyte concentration value from color formation of the test field (134), wherein the method comprises:
a) performing the adjustment method (122) according to any one of the preceding claims;
b) carrying out, by at least one individual user (113), a plurality of analyte measurements, wherein the analyte measurements, at least partly, comprise capturing images of at least a part of an optical test strip (132) having a test field (134) by using the camera (116), thereby obtaining user-specific training data on the analyte measurements;
c) analyzing the user-specific training data obtained in step b) (142) and assigning the individual user (113) to at least one individual user profile of the user profiles; and
d) providing user profile-specific measurement setup adjustments for the individual user profile.

6. The analytical method (120) according to the preceding claim, wherein the method further comprises, before step b) (142):
e) assigning the individual user (113) to a predefined starting profile.

7. The analytical method (120) according to any one of the preceding claims referring to an analytical method (120), wherein the analytical method (120) comprises transmitting the user-specific training data obtained in step b) (142) from the individual user's mobile device (112) to at least one evaluation server device (124), wherein steps c) (144) and d) (146), at least partially, are performed by the evaluation server device (124).

8. The analytical method (120) according to the preceding claim, wherein the user profile-specific measurement setup adjustments for the individual user profile are transmitted from the evaluation server device (124) to the individual user's mobile device (112).

9. The analytical method (120) according to the preceding claim, further comprising:
f) carrying out, by the individual user (113) using the individual user's mobile device (112), at least one analyte measurement, wherein the analyte measurement, at least partly, comprise capturing at least one image of at least a part of an optical test strip (132) having a test field (134) by using the camera (116), and evaluating the at least one image for deriving at least one measurement value of the concentration of the analyte in the bodily fluid, wherein the analyte measurement is performed by using the user profile-specific measurement setup adjustments for the individual user (113).

10. The analytical method (120) according to any one of the preceding claims referring to an analytical method (120), wherein step c) (144) comprises using at least one self-learning algorithm.

11. An adjustment system (110) for performing the method according to any one of claims 1 to 4, the adjustment system (110) comprising:
I) a receiving device (126) for receiving training data on analytical measurements, the training data being obtained by a plurality of users (114) carrying out a plurality of analyte measurements, wherein the analyte measurements, at least partly, comprise capturing images of at least a part of an optical test strip (132) having a test field (134) by using the camera (116), thereby obtaining training data on the analyte measurements;
II) an evaluation server device (124) configured for performing step ii) (138) of the adjustment method (122) according to any one of claims 1 to 4; and
III) a transmission device (128) for transmitting profile-specific measurement setup adjustments provided in step ii) (138).

12. A mobile device (112) having at least one camera (116), the mobile device (112) being configured for:
- performing step b) (142) of the analytical method (120) according to any one of claims 5 to 10;
- receiving the user profile-specific measurement setup adjustments for the individual user profile provided in step d) of claim 5 (146);
wherein the mobile device carries out step f) of claim 9.

13. A computer program comprising instructions which, when the program is executed by a mobile device (112) having a camera (116), cause the mobile device (112) to carry out at least method steps b) (142) and f) (150) of the analytical method (120) of claim 9.

14. A computer-readable storage medium comprising instructions which, when executed by a mobile device (112) having a camera (116), cause the mobile device (112) to carry out at least method steps b) (142) and f) (150) of the analytical method (150) of claim 9.

15. A computer program comprising instructions which, when the program is executed by the adjustment system (110) according to claim 11, cause the adjustment system (110) to carry out the adjustment method (122) of any one of claims 1 to 4.

16. A computer-readable storage medium comprising instructions which, when executed by the adjustment system (110) according to claim 11, cause the adjustment system (110) to carry out the method of any one of claims 1 to 4.

## Patentansprüche

1. Einstellungsverfahren (122) zum Einstellen einer Messeinrichtung für ein Analyseverfahren (120) zum Bestimmen einer Konzentration eines Analyten in einer Körperflüssigkeit, wobei das Analyseverfahren (120) das Verwenden einer mobilen Vorrichtung (112) mit einer Kamera (116) umfasst, wobei das Analyseverfahren (120) ferner das Aufnehmen mindestens eines Bildes von mindestens einem Teil eines optischen Teststreifens (132) mit einem Testfeld (134) unter Verwendung der Kamera (116) umfasst und wobei das Analyseverfahren (120) ferner das Bestimmen mindestens eines Analytkonzentrationswertes anhand der Farbbildung des Testfeldes (134) umfasst, wobei das Einstellungsverfahren (122) Folgendes umfasst:
i) Durchführen einer Vielzahl von Analytmessungen durch eine Vielzahl von Benutzern (114), wobei die Analytmessungen mindestens teilweise das Aufnehmen von Bildern von mindestens einem Teil eines optischen Teststreifens (132) mit einem Testfeld (134) unter Verwendung der Kamera (116) umfassen,
**dadurch gekennzeichnet, dass**:
dadurch Trainingsdaten zu den Analytmessungen erhalten werden, wobei die Trainingsdaten mindestens eines von Folgendem umfassen:
- Farbinformationen, die aus den Bildern gewonnen werden;
- Informationen, die aus mindestens einer Farbreferenzkarte gewonnen werden, die in den Bildern sichtbar ist;
- Analytmesswerte, die aus den Bildern gewonnen werden;
- Sensordaten, die unter Verwendung mindestens eines Sensors der mobilen Vorrichtungen (112) der Benutzer erhalten werden, der aus der Gruppe ausgewählt ist, die aus Folgendem besteht: einem Winkelsensor; einem Lichtsensor; einem Bewegungssensor; einem Beschleunigungssensor; einem Kreiselsensor; einem Magnetsensor; einem GPS-Sensor; einem Drucksensor; einem Temperatursensor; einem biometrischen Sensor;
- Einrichtungsinformationen bezüglich einer Einrichtung der mobilen Vorrichtungen (112) der Benutzer zum Durchführen der Analytmessungen;
- Gesundheitsinformationen bezüglich der Benutzer;
ii) Analysieren der in Schritt i) (136) erhaltenen Trainingsdaten, wodurch Ähnlichkeiten bei den Trainingsdaten identifiziert werden und eine Vielzahl von Benutzerprofilen gemäß den Ähnlichkeiten bei den Trainingsdaten identifiziert werden, wobei jeder Satz von Trainingsdaten eine Variable umfasst, wobei die Ähnlichkeiten von zwei Sätzen von Trainingsdaten von den Werten der jeweiligen Variablen, die in einem speziellen Bereich oder um nicht mehr als eine vorgegebene Schwelle voneinander entfernt liegen, definiert werden, wobei sich die in Schritt ii) (138) identifizierten Ähnlichkeiten mindestens teilweise auf mindestens eines von Folgendem beziehen:
- Lichtbedingungen beim Durchführen von Schritt i);
- den Tremor der Benutzer beim Durchführen von Schritt i);
wobei das Benutzerprofil mindestens ein Informationsdetail zu dem Benutzer in Bezug auf eines oder mehreres von Folgendem umfasst: einen körperlichen Zustand des Benutzers; eine Gewohnheit des Benutzers; einen Erfahrungsgrad des Benutzers; eine persönliche Situation des Benutzers; und
iii) Bereitstellen von profilspezifischen Messeinrichtungseinstellungen für mindestens eines der Benutzerprofile, wobei sich die profilspezifischen Messeinrichtungseinstellungen auf mindestens ein spezielles Umfeld zum Durchführen der mindestens einen Analytmessung von dem Benutzer beziehen, wobei der Benutzer zu einem speziellen Benutzerprofil gehört, wobei sich die profilspezifischen Messeinrichtungseinstellungen mindestens teilweise auf eines oder mehreres von Folgendem beziehen:
- Kameraeinstellungen beim Durchführen von Schritt i), wenn sich die in Schritt ii) identifizierten Ähnlichkeiten mindestens teilweise auf Lichtbedingungen beim Durchführen von Schritt i) beziehen;
- einen zulässigen Bewegungsbereich der mobilen Vorrichtung (112) beim Durchführen von Schritt i), wenn sich die in Schritt ii) identifizierten Ähnlichkeiten mindestens teilweise auf den Tremor der Benutzer beim Durchführen von Schritt i) beziehen;
- Bildunschärfe der in Schritt i) aufgenommenen Bilder, wenn sich die in Schritt ii) identifizierten Ähnlichkeiten mindestens teilweise auf den Tremor der Benutzer beim Durchführen von Schritt i) beziehen.

2. Einstellungsverfahren (122) nach dem vorstehenden Anspruch, wobei Schritt ii) (138) das Verwenden mindestens eines selbstlernenden Algorithmus umfasst.

3. Einstellungsverfahren (122) nach einem der vorstehenden Ansprüche, wobei das Einstellungsverfahren (122) das Übertragen der in Schritt i) (136) erhaltenen Trainingsdaten aus den mobilen Vorrichtungen (112) der Benutzer auf mindestens eine Auswerteservervorrichtung (124) umfasst, wobei mindestens Schritt ii) (138) von der Auswerteservervorrichtung (124) durchgeführt wird.

4. Einstellungsverfahren (122) nach einem der vorstehenden Ansprüche, wobei sich die profilespezifischen Messeinrichtungseinstellungen mindestens teilweise auf mindestens eines von Folgendem beziehen:
- eine Handhabungsprozedur der Analytmessung;
- eine Hardware-Einrichtung der mobilen Vorrichtung (112);
- eine Software-Einrichtung der mobilen Vorrichtung (112);
- Anweisungen, die die mobile Vorrichtung (112) dem Benutzer gibt;
- einen Verlässlichkeitsgrad des Messergebnisses der Analytmessung;
- einen Toleranzbereich für zulässige Parameter beim Durchführen der Analytmessung;
- eine zeitliche Abfolge für das Durchführen der Analytmessung;
- einen Sicherheitsalgorithmus für das Durchführen der Analytmessung;
- eine bessere Analytmessungsgenauigkeit.

5. Analyseverfahren (120) zum Bestimmen einer Konzentration eines Analyten in einer Körperflüssigkeit, wobei das Verfahren das Verwenden einer mobilen Vorrichtung (112) mit einer Kamera (116) umfasst, wobei das Verfahren das Aufnehmen mindestens eines Bildes von mindestens einem Teil eines optischen Teststreifens (132) mit einem Testfeld (134) unter Verwendung der Kamera (116) umfasst und wobei das Verfahren ferner das Bestimmen mindestens eines Analytkonzentrationswertes anhand der Farbbildung des Testfeldes (134) umfasst, wobei das Verfahren Folgendes umfasst:
a) Durchführen des Einstellungsverfahrens (122) nach einem der vorstehenden Ansprüche;
b) Durchführen einer Vielzahl von Analytmessungen von mindestens einem einzelnen Benutzer (113), wobei die Analytmessungen mindestens teilweise das Aufnehmen von Bildern von mindestens einem Teil eines optischen Teststreifens (132) mit einem Testfeld (134) unter Verwendung der Kamera (116) umfassen, wodurch benutzerspezifische Trainingsdaten zu den Analytmessungen erhalten werden;
c) Analysieren der in Schritt b) (142) erhaltenen benutzerspezifischen Trainingsdaten und Zuordnen des einzelnen Benutzers (113) zu mindestens einem einzelnen Benutzerprofil von den Benutzerprofilen und
d) Bereitstellen benutzerprofilspezifischer Messeinrichtungseinstellungen für das einzelne Benutzerprofil.

6. Analyseverfahren (120) nach dem vorstehenden Anspruch, wobei das Verfahren ferner vor Schritt b) (142) Folgendes umfasst:
e) Zuordnen des einzelnen Benutzers (113) zu einem vordefinierten Startprofil.

7. Analyseverfahren (120) nach einem der vorstehenden Ansprüche, die sich auf ein Analyseverfahren (120) beziehen, wobei das Analyseverfahren (120) das Übertragen der in Schritt b) (142) erhaltenen benutzerspezifischen Trainingsdaten aus der mobilen Vorrichtung (112) des einzelnen Benutzers auf mindestens eine Auswerteservervorrichtung (124) umfasst, wobei Schritte c) (144) und d) (146) mindestens teilweise von der Auswerteservervorrichtung (124) durchgeführt werden.

8. Analyseverfahren (120) nach dem vorstehenden Anspruch, wobei die benutzerprofilspezifischen Messeinrichtungseinstellungen für das einzelne Benutzerprofil aus der Auswerteservervorrichtung (124) auf die mobile Vorrichtung (112) des einzelnen Benutzers übertragen werden.

9. Analyseverfahren (120) nach dem vorstehenden Anspruch, ferner umfassend:
f) Durchführen mindestens einer Analytmessung von dem einzelnen Benutzer (113) unter Verwendung der mobilen Vorrichtung (112) des einzelnen Benutzers, wobei die Analytmessung mindestens teilweise das Aufnehmen mindestens eines Bildes von mindestens einem Teil eines optischen Teststreifens (132) mit einem Testfeld (134) unter Verwendung der Kamera (116) und das Auswerten des mindestens einen Bildes umfasst, um mindestens einen Messwert der Konzentration des Analyten in der Körperflüssigkeit abzuleiten, wobei die Analytmessung unter Verwendung der benutzerprofilspezifischen Messeinrichtungseinstellungen für den einzelnen Benutzer (113) durchgeführt wird.

10. Analyseverfahren (120) nach einem der vorstehenden Ansprüche, die sich auf ein Analyseverfahren (120) beziehen, wobei Schritt c) (144) das Verwenden mindestens eines selbstlernenden Algorithmus umfasst.

11. Einstellungssystem (110) zum Durchführen des Verfahrens nach einem der Ansprüche 1 bis 4, wobei das Einstellungssystem (110) Folgendes umfasst:
I) eine Empfangsvorrichtung (126) zum Empfangen von Trainingsdaten zu analytischen Messungen, wobei die Trainingsdaten von einer Vielzahl von Benutzern (114) erhalten werden, die eine Vielzahl von Analytmessungen durchführen, wobei die Analytmessungen mindestens teilweise das Aufnehmen von Bildern von mindestens einem Teil eines optischen Teststreifens (132) mit einem Testfeld (134) unter Verwendung der Kamera (116) umfassen, wodurch Trainingsdaten zu den Analytmessungen erhalten werden;
II) eine Auswerteservervorrichtung (124), die zum Durchführen von Schritt ii) (138) des Einstellungsverfahrens (122) nach einem der Ansprüche 1 bis 4 ausgebildet ist, und
III) eine Übertragungsvorrichtung (128) zum Übertragen profilspezifischer Messeinrichtungseinstellungen, die in Schritt ii) (138) bereitgestellt werden.

12. Mobile Vorrichtung (112) mit mindestens einer Kamera (116), wobei die mobile Vorrichtung (112) zu Folgendem ausgebildet ist:
- Durchführen von Schritt b) (142) des Analyseverfahrens (120) nach einem der Ansprüche 5 bis 10;
- Empfangen der benutzerprofilspezifischen Messeinrichtungseinstellungen für das einzelne Benutzerprofil, das in Schritt d) von Anspruch 5 (146) bereitgestellt wird;
wobei die mobile Vorrichtung Schritt f) von Anspruch 9 durchführt.

13. Computerprogramm, umfassend Anweisungen, die bei der Ausführung des Programms von einer mobilen Vorrichtung (112) mit einer Kamera (116) die mobile Vorrichtung (112) veranlassen, mindestens Verfahrensschritte b) (142) und f) (150) des Analyseverfahrens (120) von Anspruch 9 durchzuführen.

14. Computerlesbares Speichermedium, umfassend Anweisungen, die bei der Ausführung von einer mobilen Vorrichtung (112) mit einer Kamera (116) die mobile Vorrichtung (112) veranlassen, mindestens Verfahrensschritte b) (142) und f) (150) des Analyseverfahrens (150) von Anspruch 9 durchzuführen.

15. Computerprogramm, umfassend Anweisungen, die bei der Ausführung des Programms von dem Einstellungssystem (110) nach Anspruch 11 das Einstellungssystem (110) veranlassen, das Einstellungsverfahren (122) nach einem der Ansprüche 1 bis 4 durchzuführen.

16. Computerlesbares Speichermedium, umfassend Anweisungen, die bei der Ausführung von dem Einstellungssystem (110) nach Anspruch 11 das Einstellungssystem (110) veranlassen, das Verfahren nach einem der Ansprüche 1 bis 4 durchzuführen.

## Revendications

1. Procédé de réglage (122) permettant de régler une configuration de mesure pour un procédé analytique (120) permettant de déterminer une concentration d'un analyte dans un fluide corporel, le procédé analytique (120) comprenant l'utilisation d'un dispositif mobile (112) ayant une caméra (116), le procédé analytique (120) comprenant en outre la capture d'au moins une image d'au moins une partie d'une bandelette de test optique (132) ayant un champ de test (134) en utilisant la caméra (116), et dans lequel le procédé analytique (120) comprend en outre la détermination d'au moins une valeur de concentration d'analyte à partir d'une formation de couleur du champ de test (134), dans lequel le procédé de réglage (122) comprend :
i) la réalisation, par une pluralité d'utilisateurs (114), d'une pluralité de mesures d'analyte, les mesures d'analyte comprenant, au moins en partie, la capture d'images d'au moins une partie d'une bandelette de test optique (132) ayant un champ de test (134) en utilisant la caméra (116),
**caractérisé par** :
l'obtention de ce fait de données d'entraînement sur les mesures d'analyte,
les données d'entraînement comprenant au moins l'un parmi :
- des informations de couleurs dérivées des images ;
- des informations dérivées d'au moins une carte de référence de couleurs visible dans les images ;
- des valeurs de mesures d'analyte dérivées des images ;
- des données de capteurs obtenues en utilisant au moins un capteur des dispositifs mobiles (112) des utilisateurs choisi dans le groupe constitué par : un capteur d'angle ; un capteur de luminosité ; un capteur de mouvement ; un capteur d'accélération ; un capteur gyroscopique ; un capteur magnétique ; un capteur GPS ; un capteur de pression ; un capteur de température ; un capteur biométrique ;
- des informations de configuration relatives à une configuration des dispositifs mobiles (112) des utilisateurs pour réaliser les mesures d'analyte ;
- des informations de santé relatives aux utilisateurs ;
ii) l'analyse des données d'entraînement obtenues à l'étape i) (136), identifiant de ce fait des similarités dans les données d'entraînement et identifiant une pluralité de profils d'utilisateurs en fonction des similarités dans les données d'entraînement, dans lequel chaque ensemble de données d'entraînement comprend une variable, dans lequel les similarités de deux ensembles de données d'entraînement sont définies par les valeurs des variables respectives se trouvant dans une plage spécifique ou étant espacées de pas plus d'un seuil donné, dans lequel les similarités identifiées à l'étape ii) (138) se rapportent au moins en partie à au moins l'un de ce qui suit :
- des conditions d'éclairage lors de la mise en oeuvre de l'étape i) ;
- au tremblement des utilisateurs lors de la mise en oeuvre de l'étape i) ;
dans lequel le profil d'utilisateur comprend au moins un élément d'informations sur l'utilisateur relatif à un ou plusieurs parmi : un état physique de l'utilisateur ; une habitude de l'utilisateur ; un degré d'expérience de l'utilisateur ; une situation personnelle de l'utilisateur ; et
iii) la fourniture de réglages de configuration de mesure spécifiques à un profil pour au moins l'un des profils d'utilisateurs, les réglages de configuration de mesure spécifiques à un profil se rapportant à au moins un réglage spécifique pour réaliser l'au moins une mesure d'analyte par l'utilisateur, l'utilisateur appartenant à un profil d'utilisateur spécifique, les réglages de configuration de mesure spécifiques à un profil se rapportant au moins en partie à un ou plusieurs parmi :
- les réglages de caméra lors de la réalisation de l'étape i) au cas où les similarités identifiées à l'étape ii) se rapportent au moins en partie à des conditions d'éclairage lors de la mise en oeuvre de l'étape i) ;
- une plage admissible de mouvement du dispositif mobile (112) lors de la mise en oeuvre de l'étape i) au cas où les similarités identifiées à l'étape ii) se rapportent au moins en partie à un tremblement de l'utilisateur lors de la mise en oeuvre de l'étape i) ;
- un flou d'image des images capturées à l'étape i) au cas où les similarités identifiées à l'étape ii) se rapportent au moins en partie à un tremblement des utilisateurs lors de la mise en oeuvre de l'étape i).

2. Procédé de réglage (122) selon la revendication précédente, dans lequel l'étape ii) (138) comprend l'utilisation d'au moins un algorithme d'auto-apprentissage.

3. Procédé de réglage (122) selon l'une quelconque des revendications précédentes, dans lequel le procédé de réglage (122) comprend la transmission des données d'entraînement obtenues à l'étape i) (136) des dispositifs mobiles (112) des utilisateurs vers au moins un dispositif serveur d'évaluation (124), dans lequel au moins l'étape ii) (138) est mise en oeuvre par le dispositif serveur d'évaluation (124).

4. Procédé de réglage (122) selon l'une quelconque des revendications précédentes, dans lequel les réglages de configuration de mesure spécifiques à un profil se rapportent au moins en partie à au moins l'un parmi :
- une procédure de manipulation de la mesure d'analyte ;
- une configuration de matériel du dispositif mobile (112) ;
- une configuration de logiciel du dispositif mobile (112) ;
- des instructions données par le dispositif mobile (112) à l'utilisateur ;
- un degré de fiabilité de résultat de mesure de la mesure d'analyte ;
- une plage de tolérance pour les paramètres admissibles lors de la mise en oeuvre de la mesure d'analyte ;
- une séquence temporelle pour la mise en oeuvre de la mesure d'analyte ;
- un algorithme à sécurité intégrée pour la mise en oeuvre de la mesure d'analyte ;
- une précision de mesure d'analyte améliorée.

5. Procédé analytique (120) permettant de déterminer une concentration d'un analyte dans un fluide corporel, le procédé comprenant l'utilisation d'un dispositif mobile (112) ayant une caméra (116), dans lequel le procédé comprend la capture d'au moins une image d'au moins une partie d'une bandelette de test optique (132) ayant un champ de test (134) en utilisant la caméra (116), et dans lequel le procédé comprend en outre la détermination d'au moins une valeur de concentration d'analyte à partir d'une formation de couleur du champ de test (134), dans lequel le procédé comprend :
a) la mise en oeuvre du procédé de réglage (122) selon l'une quelconque des revendications précédentes ;
b) la réalisation, par au moins un utilisateur individuel (113), d'une pluralité de mesures d'analyte, les mesures d'analyte comprenant, au moins en partie, la capture d'images d'au moins une partie d'une bandelette de test optique (132) ayant un champ de test (134) en utilisant la caméra (116), obtenant de ce fait des données d'entraînement spécifiques à un utilisateur sur les mesures d'analyte ;
c) l'analyse des données d'entraînement spécifiques à l'utilisateur obtenues à l'étape b) (142) et l'affectation de l'utilisateur individuel (113) à au moins un profil d'utilisateur individuel parmi les profils d'utilisateurs ; et
d) la fourniture de réglages de configuration de mesure spécifiques au profil d'utilisateur pour le profil d'utilisateur individuel.

6. Procédé analytique (120) selon la revendication précédente, dans lequel le procédé comprend en outre, avant l'étape b) (142) :
e) l'affectation de l'utilisateur individuel (113) à un profil de départ prédéfini.

7. Procédé analytique (120) selon l'une quelconque des revendications précédentes se rapportant à un procédé analytique (120), dans lequel le procédé analytique (120) comprend la transmission des données d'entraînement spécifiques à l'utilisateur obtenues à l'étape b) (142) du dispositif mobile (112) d'un utilisateur individuel vers au moins un dispositif serveur d'évaluation (124), dans lequel les étapes c) (144) et d) (146), au moins en partie, sont mises en oeuvre par le dispositif serveur d'évaluation (124).

8. Procédé analytique (120) selon la revendication précédente, dans lequel les réglages de configuration de mesure spécifiques au profil d'utilisateur pour le profil d'utilisateur individuel sont transmis du dispositif de serveur d'évaluation (124) au dispositif mobile (112) d'un utilisateur individuel.

9. Procédé analytique (120) selon la revendication précédente, comprenant en outre :
f) la réalisation, par l'utilisateur individuel (113) en utilisant le dispositif mobile (112) d'un utilisateur individuel, d'au moins une mesure d'analyte, la mesure d'analyte comprenant, au moins en partie, la capture d'au moins une image d'au moins une partie d'une bandelette de test optique (132) ayant un champ de test (134) en utilisant la caméra (116), et l'évaluation de l'au moins une image pour en déduire au moins une valeur de mesure de la concentration de l'analyte dans le fluide corporel, la mesure d'analyte étant mise en oeuvre en utilisant les réglages de configuration de mesure spécifiques au profil d'utilisateur pour l'utilisateur individuel (113).

10. Procédé analytique (120) selon l'une quelconque des revendications précédentes se rapportant à un procédé analytique (120), dans lequel l'étape c) (144) comprend l'utilisation d'au moins un algorithme d'auto-apprentissage.

11. Système de réglage (110) permettant de mettre en oeuvre le procédé selon l'une quelconque des revendications 1 à 4, le système de réglage (110) comprenant :
I) un dispositif de réception (126) permettant de recevoir des données d'entraînement sur les mesures analytiques, les données d'entraînement étant obtenues par une pluralité d'utilisateurs (114) réalisant une pluralité de mesures d'analyte, les mesures d'analyte comprenant, au moins en partie, la capture d'images d'au moins une partie d'une bandelette de test optique (132) ayant un champ de test (134) en utilisant la caméra (116), obtenant de ce fait des données d'entraînement sur les mesures d'analyte ;
II) un dispositif serveur d'évaluation (124) configuré pour mettre en oeuvre l'étape ii) (138) du procédé de réglage (122) selon l'une quelconque des revendications 1 à 4 ; et
III) un dispositif de transmission (128) permettant de transmettre les réglages de configuration de mesure spécifiques au profil fournis à l'étape ii) (138).

12. Dispositif mobile (112) ayant au moins une caméra (116), le dispositif mobile (112) étant configuré pour :
- mettre en oeuvre l'étape b) (142) du procédé analytique (120) selon l'une quelconque des revendications 5 à 10 ;
- recevoir les réglages de configuration de mesure spécifiques au profil d'utilisateur pour le profil d'utilisateur individuel fournis à l'étape d) de la revendication 5 (146) ;
dans lequel le dispositif mobile réalise l'étape f) de la revendication 9.

13. Programme informatique comprenant des instructions qui, lorsque le programme est exécuté par un dispositif mobile (112) ayant une caméra (116), amènent le dispositif mobile (112) à réaliser au moins les étapes de procédé b) (142) et f) (150) du procédé analytique (120) selon la revendication 9.

14. Support de stockage lisible par ordinateur comprenant des instructions qui, lorsqu'elles sont exécutées par un dispositif mobile (112) ayant une caméra (116), amènent le dispositif mobile (112) à réaliser au moins les étapes de procédé b) (142) et f) (150) du procédé analytique (150) selon la revendication 9.

15. Programme informatique comprenant des instructions qui, lorsque le programme est exécuté par le système de réglage (110) selon la revendication 11, amènent le système de réglage (110) à réaliser le procédé de réglage (122) selon l'une quelconque des revendications 1 à 4.

16. Support de stockage lisible par ordinateur comprenant des instructions qui, lorsqu'elles sont exécutées par le système de réglage (110) selon la revendication 11, amènent le système de réglage (110) à réaliser le procédé selon l'une quelconque des revendications 1 à 4.
